# EUROPEAN PATENT SPECIFICATION

(11) **EP 1 162 463 B1**
(45) Date of publication and mention of the grant of the patent: **27.08.2003**
(21) Application number: 01114149.6
(22) Date of filing: 11.06.2001
(51) Int. Cl.: G01N 33/86, B01L 3/14, G01N 33/49

(54) **Collection device for blood**
Sammelvorrichtung für Blut
Dispositif de récuperation du sang

(30) Priority: 10.06.2000 US 210685 P
(43) Date of publication of application: 12.12.2001
(73) Proprietor: Becton, Dickinson and Company, Franklin Lakes, New Jersey 07417 (US)
(72) Inventor: Harrop, Andrew John, Okehampton, Devon EX20 4PS (GB); Dubrowny, Nancy, Garfield, New Jersey 07026 (US)
(74) Representative: von Kreisler, Alek, Dipl.-Chem.

(56) References cited:
- EP-A- 0 073 551
- EP-A- 0 628 816

## Description

### 1. Field of Invention

The present invention relates to a method of promoting clotting of a blood sample in a container comprising a multi-layer coating composition, a container for promoting rapid clotting of a blood sample, a method for making said container and a receptacle comprising said container.

### 2. Description of Related Art

Blood samples are routinely taken in evacuated collection tubes. One end of a double-ended needle is inserted into a patient's vein. The other end of the needle then punctures a septum covering the open end of the tube so that the vacuum in the tube draws the blood sample through the needle into the tube. Using this technique, a plurality of samples can be taken using a single needle puncture of the skin.

Blood collected in evacuated tubes often must be clotted prior to clinical examination. It is desirable to form a dense clot as rapidly and completely as possible to facilitate clean separation of the clot from the serum layer by centrifugation. To achieve this, collection tubes frequently employ a clot activator. Typical activators are diatomaceous earth and particles of inorganic silicates, or biochemicals such as ellagic acid, thrombin and thromboplastin.

Two types of activators, classified in the art according to the portion of the blood coagulation cascade stimulated, are conventionally employed. Particulate activators share a common biochemical mechanism of clot activation known as contact activation of the intrinsic pathway. Whole blood contains all of the necessary factors to cause clotting by the intrinsic pathway. Clot activation by the intrinsic pathway is surface area dependent, i.e. the time required to form a complete blood clot is dependent on the total number of activating surface sites per unit area on the activator surface relative to the volume of blood. Greater surface area, provided by finely divided particulate activators, leads to shorter clot times. Particulate activators are used in practically all commercial blood collection tubes and lead to dense, crosslinked clots that cleanly separate from the serum in a centrifuge. Clot formation, however, is relatively slow, and about 30-60 minutes are required prior to centrifugation. Typical particulate activators used commercially are silica impregnated in fabric, silica particles in small plastic cups or silicate particles applied to the tube wall in polyvinylpyrrolidone (PVP).

The second type of clot activators induces clotting through a different part of the coagulation cascade known in the art as the extrinsic pathway. The extrinsic system relies on the presence of a substance not normally present in whole blood. Activation is biochemical in nature and is concentration dependent. Clot activation rates lead to clot formation in 10-20 minutes, but clots resulting from the extrinsic pathway are gelatinous in nature and do not cleanly separate from serum.

EP-A-0628816 discloses a blood collection container containing a clotting enhancer which activated both the intrinsic and extrinsic pathways. The activators for intrinsic and extrinsic coagulation are applies to separate areas of the wall surface of the container. Alternatively, glass microspheres coated with an immobilized activator of the extrinsic coagulation pathway may be combined with unmodified microspheres as activators of the intrinsic pathway. Preferably the coated microspheres may be crushed whereby the native unmodified glass surface is exposed and the ratio of surface areas of intrinsic and extrinsic activating surfaces is 50/50.

EP-A-0073551 discloses a blood collection container equipped with various reagents (e.g. coagulation activators) applied to pieces of substrate, e.g. paper. Potential disadvantages of the introduction of reagents into containers by coating of the internal surfaces are mentioned.

There is a need for a blood collection tube with means for promoting clot acceleration of a blood sample which provides an enhanced rate of blood coagulation but which does not remain in the serum layer or become part of the clot or centrifugation, thus avoiding potential interference with clinical tests and which is impermeable to moisture ingress.

### SUMMARY OF THE INVENTION

The present invention is a container with means for promoting clot acceleration of a blood sample. The container comprises a multi-layer coating of an intrinsic coagulation activator and an extrinsic activator.

In particular the present invention relates to a container for promoting clotting of a blood sample obtainable by applying a layer of an intrinsic coagulation activator to an interior surface of the container, applying an extrinsic coagulation activator (36) onto the layer of the intrinsic coagulation activator.

In a further embodiment the present invention relates to a method of promoting clotting of a blood sample in a container by activating the intrinsic and extrinsic pathways, comprising applying a layer of an intrinsic coagulation activator to an interior surface of the container, applying an extrinsic coagulation activator onto the layer of the intrinsic coagulation activator.

Finally the present invention relates to a moisture impermeable receptacle comprising the container of the invention and a moisture absorbing material.

Preferred embodiments of the invention become evident from the dependent claims.

Preferably, the intrinsic coagulation activator is diatomaceous earth or ground silica. Most preferably, the intrinsic coagulation activator is ground silica.

Desirably, the extrinsic coagulation activator is ellagic acid or a protein. Preferably, the extrinsic coagulation activator is thrombin, heparinase or fibrinogen. Most preferably, the extrinsic coagulation activator is thrombin.

Desirably, the means for promoting clot acceleration is applied inside a container as follows:
(a) mixing polyvinylpyrrolidene (PVP) and an intrinsic coagulation activator in water to form a first mixture;
(b) spraying the first mixture onto the inner surface of the container;
(c) drying the first mixture;
(d) mixing an extrinsic coagulation activator with water to form a second mixture;
(e) spraying the second mixture onto the first mixture; and
(f) drying the second mixture.

Preferably, the means for promoting clot acceleration is applied inside a container as follows:
(a) mixing a water soluble surfactant with water to form a first mixture;
(b) spraying the first mixture onto the inner surface of the container;
(c) drying the first mixture;
(d) mixing polyvinylpyrrolidene (PVP) and an intrinsic coagulation activator in water to form a second mixture;
(e) spraying the second mixture onto the first mixture;
(f) drying the second mixture;
(g) mixing an extrinsic coagulation activator with water to form a third mixture;
(h) spraying the third mixture onto the second mixture; and
(i) drying the third mixture.

Most preferably, the means for promoting clot acceleration is applied inside a container as follows:
(a) mixing a water soluble silicone surfactant with water to form a first mixture;
(b) spraying the first mixture onto the inner surface of the container;
(c) drying the first mixture;
(d) mixing polyvinylpyrrolidiene (PVP) and ground silica in water to form a second mixture;
(e) spraying the second mixture onto the first mixture;
(f) drying the second mixture;
(g) mixing thrombin with water to form a third mixture;
(h) spraying the third mixture onto the second mixture; and
(i) drying the third mixture.

In addition, the container with the intrinsic and extrinsic activators is provided in a moisture impermeable receptacle with a moisture absorbing material. The moisture impermeable receptacle with a moisture absorbing material maintains the integrity of the thrombin in the container. Since thrombin may degrade when exposed to high external humidity, the integrity for promoting clot acceleration of a blood sample with thrombin is found to be maintained in a moisture impermeable receptacle.

Most notably, the present invention provides the means for promoting clot acceleration of a blood sample at a faster rate than when only silica is used.

In addition, the present invention provides the means for promoting clot acceleration of a blood sample even when exposed to high humidity.

### DESCRIPTION OF THE DRAWINGS

FIG. 1 is a perspective view of a collection assembly.
FIG. 2 is a cross-sectional view of the assembly of FIG. 1 illustrating the multi-layer coatings of the present invention.
FIG. 3 is a perspective view of the assembly of FIGS. 1 and 2 in a pouch assembly.

### DETAILED DESCRIPTION

The present invention may be embodied in other specific forms and is not limited to any specific embodiment described in detail which is merely exemplary. Various other modifications will be apparent to and readily made by those skilled in the art without departing from the scope and spirit of the invention. The scope of the invention will be measured by the appended claims and their equivalents.

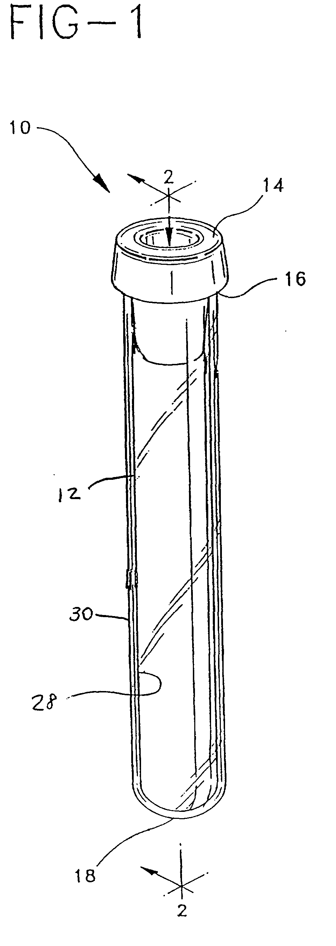

Referring to the drawings in which like reference characters refer to like parts throughout the several views thereof in FIG. 1, shows a collection assembly **10** comprising a sidewall **12** extending from an open end **16** to a bottom closed end **18** and a closure **14**. Sidewall **12** further includes an inner wall surface **28** and an outer wall surface **30**.

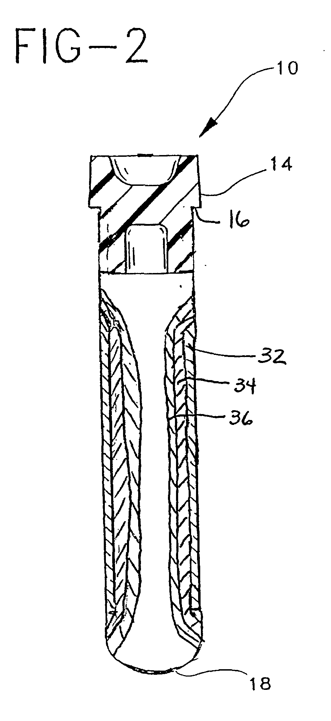

As shown in FIG. 2, the multi-layer coating of the present invention is located on inner wall surface **28** of the assembly **10**. The multi-layer coating comprises a first layer **32** of a surfactant, a second layer **34** of an intrinsic coagulation activator and a third layer **36** of an extrinsic activator.

First layer **32** comprises a water soluble silicone surfactant. Second layer **34** comprises ground silica and third layer **36** comprises thrombin.

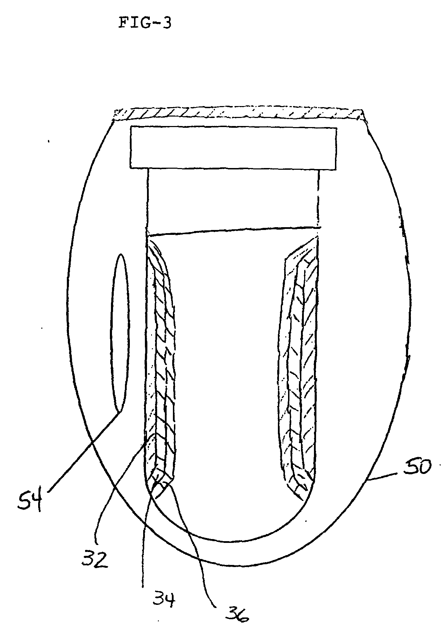

FIG. 3 shows the collection assembly in a pouch **50**. Within pouch **50** is a moisture absorbing material **54**.

Collection assembly **10** is preferably of plastic, but may be of glass. Suitable plastics are polyvinyl chloride, polypropylene (PP) and polyethylene terephthalate (PET).

If the collection assembly is impermeable to moisture transmission, then a pouch assembly is not necessary. However, when moisture penetration is a concern, the collection assembly may be placed within pouch **50**.

It was found during testing of the present plastic collection assembly that when thrombin was used as an extrinsic activator, high levels of humidity caused degradation of the thrombin. Moisture penetrating the plastic receptacle walls was determined to be the causative agent. Thus, the present plastic collection device is most preferably packaged within an impermeable pouch containing a moisture absorbing material.

Pouch **50** is preferably of an aluminum foil laminated with LDPE/OPET/adhesive/sealant. LDPE/OPET is a low density polyethylene/oriented polyethylene terephthalate. The layering provides mechanical protection of the foil, heat sealing and opening features. Pouch **50** is preferably packaged with moisture absorbing material **54** (desiccant) which may be of silica dioxide (silica gel) or moisture absorbing beads, rods, sheets, or sachets and which may incorporate warning colors and/or wording which appear when the moisture content exceeds a certain level. Pouch **50** may accommodate one or more collection assemblies.

### Example 1

This example demonstrates the improved clotting activity when the present invention is utilized.

Fifteen (15) plastic tubes coated with a soluble silicone surfactant, PVP and ground silica and thrombin were used in this example as compared to glass control tubes that are untreated to show the comparative clotting efficacy of the present invention versus glass tubes.

Blood was drawn into the tubes and the glass control tube and the plastic tubes of the present invention were mixed using 10 complete inversions and allowed to clot upright in a rack at room temperature. A stopwatch started as soon as mixing was completed, which was used to document the individual clotting time for each tube. Each tube was checked for clotting every 30 seconds by picking the tube up out of the rack and tilting slightly to assess the status of the blood sample. A blood sample is considered clotted when there is no more liquid movement of the blood sample noted. At this point for each tube evaluated, the stopwatch was stopped and the time noted in seconds was considered into clotting time of that individual sample. Acceptable clotting time in seconds for the glass control tube is less than 300 seconds (5 minutes). The results of this Example are reported in Table 1.

**Table 1**

| Clotting Time in Seconds: | | |
|---|---|---|
| Donor | Glass | Plastic |
| 1 | 307 | 76 |
| 2 | 161 | 115 |
| 3 | 156 | 68 |
| 4 | 286 | 76 |
| 5 | 319 | 86 |
| 6 | 259 | 118 |
| 7 | 317 | 71 |
| 8 | 198 | 118 |
| 9 | 146 | 57 |
| 10 | 183 | 140 |
| 11 | 212 | 119 |
| 12 | 161 | 118 |
| 13 | 300 | 198 |
| 14 | 190 | 157 |
| 15 | 152 | 86 |

## Claims

1. A method of promoting clotting of a blood sample in a container by activating the intrinsic and extrinsic pathways, comprising applying a layer of an intrinsic coagulation activator to an interior surface of the container, applying an extrinsic coagulation activator onto the layer of the intrinsic coagulation activator.

2. The method of claim 1, further comprising the step of applying a layer of surfactant prior to applying the layer of the intrinsic coagulation activator.

3. The method of claim 1, wherein the extrinsic activator is selected from the group consisting of thrombin, heparinese and fibrinogen.

4. The method of claim 1, wherein the intrinsic activator is silica.

5. The method of claim 2, wherein the surfactant is a water-soluble silicone surfactant.

6. The method of claim 1, wherein the step of applying the layer of the intrinsic coagulation activator comprises mixing the intrinsic coagulation activator with polyvinylpyrrolidene, and spraying the mixture onto the inner surface, and wherein the step of applying the extrinsic coagulation activator comprises mixing the extrinsic coagulation activator with water, and spraying the mixture onto the layer of the intrinsic coagulation activator.

7. A container (10) for promoting clotting of a blood sample obtainable by applying a layer of an intrinsic coagulation activator (34) to an interior surface of the container (10), applying an extrinsic coagulation activator (36) onto the layer of the intrinsic coagulation activator (34).

8. The container of claim 7 obtainable by the further step of applying a layer of surfactant (32) prior to applying the layer of the intrinsic coagulation activator (34).

9. The container of claim 7, wherein the extrinsic activator is selected from the group consisting of thrombin, heparinese and fibrinogen.

10. The container of claim 7, wherein the intrinsic activator is silica.

11. The container of claim 8, wherein the surfactant is a water-soluble silicone surfactant.

12. The container of claim 7, wherein the step of applying the layer of the intrinsic coagulation activator (34) comprises mixing the intrinsic coagulation activator with polyvinylpyrrolidene, and spraying the mixture onto the inner surface, and wherein the step of applying the extrinsic coagulation activator comprises mixing the extrinsic coagulation activator with water, and spraying the mixture onto the layer of the intrinsic coagulation activator (34).

13. The container of claim 7 being of plastic or glass.

14. A method for making the container (10) of claim 7 comprising the steps of applying a layer of an intrinsic coagulation activator (34) to an interior surface of the container (10), applying an extrinsic coagulation activator (36) onto the layer of the intrinsic coagulation activator (34).

15. The method of claim 14, further comprising the step of applying a layer of surfactant (32) prior to applying the layer of the intrinsic coagulation activator (34).

16. The method of claim 14, wherein the extrinsic activator is selected from the group consisting of thrombin, heparinese and fibrinogen.

17. The method of claim 14, wherein the intrinsic activator is silica.

18. The method of claim 15, wherein the surfactant is a water-soluble silicone surfactant.

19. The method of claim 14, wherein the step of applying the layer of the intrinsic coagulation activator (34) comprises mixing the intrinsic coagulation activator with polyvinylpyrrolidene, and spraying the mixture onto the inner surface, and wherein the step of applying the extrinsic coagulation activator comprises mixing the extrinsic coagulation activator with water, and spraying the mixture onto the layer of the intrinsic coagulation activator (34).

20. The method of claim 14, wherein the container is of plastic or glass.

21. A moisture impermeable receptacle (50) comprising the container (10) of any one of claims 7 - 13, and a moisture absorbing material (54).

## Patentansprüche

1. Verfahren zur Förderung der Gerinnung einer Blutprobe in einem Behälter durch Aktivieren des Intrinsic- und des Extrinsic-Systems, umfassend das Auftragen einer Schicht eines intrinsischen Koagulationsaktivators auf eine Innenfläche des Behälters und das Auftragen eines extrinsischen Koagulationsaktivators auf die Schicht des intrinsischen Koagulationsaktivators.

2. Verfahren gemäß Anspruch 1, das weiterhin den Schritt des Auftragens einer Tensidschicht vor dem Auftragen der Schicht des intrinsischen Koagulationsaktivators umfasst.

3. Verfahren gemäß Anspruch 1, wobei der extrinsische Aktivator aus der Gruppe ausgewählt ist, die aus Thrombin, Heparinase und Fibrinogen besteht.

4. Verfahren gemäß Anspruch 1, wobei es sich bei dem intrinsischen Aktivator um Siliciumoxid handelt.

5. Verfahren gemäß Anspruch 2, wobei das Tensid ein wasserlösliches Silikontensid ist.

6. Verfahren gemäß Anspruch 1, wobei der Schritt des Auftragens der Schicht des intrinsischen Koagulationsaktivators das Mischen des intrinsischen Koagulationsaktivators mit Polyvinylpyrroliden und das Aufsprühen des Gemischs auf die Innenfläche umfasst und wobei der Schritt des Auftragens des extrinsischen Koagulationsaktivators das Mischen des extrinsischen Koagulationsaktivators mit Wasser und das Aufsprühen des Gemischs auf die Schicht des intrinsischen Koagulationsaktivators umfasst.

7. Behälter (10) zur Förderung der Gerinnung einer Blutprobe, erhältlich durch Auftragen einer Schicht eines intrinsischen Koagulationsaktivators (34) auf eine Innenfläche des Behälters (10) und Auftragen eines extrinsischen Koagulationsaktivators (36) auf die Schicht des intrinsischen Koagulationsaktivators (34).

8. Behälter gemäß Anspruch 7, erhältlich durch den weiteren Schritt des Auftragens einer Tensidschicht (32) vor dem Auftragen der Schicht des intrinsischen Koagulationsaktivators (34).

9. Behälter gemäß Anspruch 7, wobei der extrinsische Aktivator aus der Gruppe ausgewählt ist, die aus Thrombin, Heparinase und Fibrinogen besteht.

10. Behälter gemäß Anspruch 7, wobei es sich bei dem intrinsischen Aktivator um Siliciumoxid handelt.

11. Behälter gemäß Anspruch 8, wobei das Tensid ein wasserlösliches Silikontensid ist.

12. Behälter gemäß Anspruch 7, wobei der Schritt des Auftragens der Schicht des intrinsischen Koagulationsaktivators (34) das Mischen des intrinsischen Koagulationsaktivators mit Polyvinylpyrroliden und das Aufsprühen des Gemischs auf die Innenfläche umfasst und wobei der Schritt des Auftragens des extrinsischen Koagulationsaktivators das Mischen des extrinsischen Koagulationsaktivators mit Wasser und das Aufsprühen des Gemischs auf die Schicht des intrinsischen Koagulationsaktivators (34) umfasst.

13. Behälter gemäß Anspruch 7, der aus Kunststoff oder Glas besteht.

14. Verfahren zur Herstellung des Behälters (10) von Anspruch 7, umfassend die Schritte des Auftragens einer Schicht eines intrinsischen Koagulationsaktivators (34) auf eine Innenfläche des Behälters (10) und des Auftragens eines extrinsischen Koagulationsaktivators (36) auf die Schicht des intrinsischen Koagulationsaktivators (34).

15. Verfahren gemäß Anspruch 14, das weiterhin den Schritt des Auftragens einer Tensidschicht (32) vor dem Auftragen der Schicht des intrinsischen Koagulationsaktivators (34) umfasst.

16. Verfahren gemäß Anspruch 14, wobei der extrinsische Aktivator aus der Gruppe ausgewählt ist, die aus Thrombin, Heparinase und Fibrinogen besteht.

17. Verfahren gemäß Anspruch 14, wobei es sich bei dem intrinsischen Aktivator um Siliciumoxid handelt.

18. Verfahren gemäß Anspruch 15, wobei das Tensid ein wasserlösliches Silikontensid ist.

19. Verfahren gemäß Anspruch 14, wobei der Schritt des Auftragens der Schicht des intrinsischen Koagulationsaktivators (34) das Mischen des intrinsischen Koagulationsaktivators mit Polyvinylpyrroliden und das Aufsprühen des Gemischs auf die Innenfläche umfasst und wobei der Schritt des Auftragens des extrinsischen Koagulationsaktivators das Mischen des extrinsischen Koagulationsaktivators mit Wasser und das Aufsprühen des Gemischs auf die Schicht des intrinsischen Koagulationsaktivators (34) umfasst.

20. Verfahren gemäß Anspruch 14, wobei der Behälter aus Kunststoff oder Glas besteht.

21. Feuchtigkeitsundurchlässiges Gefäß (50), das den Behälter (10) gemäß einem der Ansprüche 7 bis 13 und ein feuchtigkeitsabsorbierendes Material (54) umfasst.

## Revendications

1. Procédé pour favoriser la coagulation d'un échantillon de sang dans un récipient en activant les voies intrinsèque et extrinsèque, comprenant l'application d'une couche d'un activateur de coagulation intrinsèque à une surface intérieure du récipient, l'application d'un activateur de coagulation extrinsèque sur la couche de l'activateur de coagulation intrinsèque.

2. Procédé selon la revendication 1, comprenant en outre l'étape qui consiste à appliquer une couche de tensioactif avant d'appliquer la couche de l'activateur de coagulation intrinsèque.

3. Procédé selon la revendication 1, dans lequel l'activateur extrinsèque est choisi dans le groupe constitué par la thrombine, l'héparinase et le fibrinogène.

4. Procédé selon la revendication 1, dans lequel l'activateur intrinsèque est la silice.

5. Procédé selon la revendication 2, dans lequel le tensioactif est un tensioactif à base de silicone soluble dans l'eau.

6. Procédé selon la revendication 1, dans lequel l'étape d'application de la couche de l'activateur de coagulation intrinsèque comprend le mélange de l'activateur de coagulation intrinsèque avec du polyvinylpyrrolidène, et la pulvérisation du mélange sur la surface interne, et dans lequel l'étape d'application de l'activateur de coagulation extrinsèque comprend le mélange de l'activateur de coagulation extrinsèque avec de l'eau, et la pulvérisation du mélange sur la couche de l'activateur de coagulation intrinsèque.

7. Récipient (10) pour favoriser la coagulation d'un échantillon de sang pouvant être obtenu par application d'une couche d'un activateur de coagulation intrinsèque (34) à une surface intérieure du récipient (10), application d'un activateur de coagulation extrinsèque (36) sur la couche de l'activateur de coagulation intrinsèque (34).

8. Récipient selon la revendication 7, pouvant être obtenu grâce à l'étape complémentaire d'application d'une couche de tensioactif (32) avant l'application de la couche de l'activateur de coagulation intrinsèque (34).

9. Récipient selon la revendication 7, dans lequel l'activateur extrinsèque est choisi dans le groupe constitué par la thrombine, l'héparinase et le fibrinogène.

10. Récipient selon la revendication 7, dans lequel l'activateur intrinsèque est la silice.

11. Récipient selon la revendication 8, dans lequel le tensioactif est un tensioactif à base de silicone soluble dans l'eau.

12. Récipient selon la revendication 7, où l'étape d'application de la couche de l'activateur de coagulation intrinsèque (34) comprend le mélange de l'activateur de coagulation intrinsèque avec du polyvinylpyrrolidène, et la pulvérisation du mélange sur la surface interne, et où l'étape d'application de l'activateur de coagulation extrinsèque comprend le mélange de l'activateur de coagulation extrinsèque avec de l'eau, et la pulvérisation du mélange sur la couche de l'activateur de coagulation intrinsèque (34).

13. Récipient selon la revendication 7, qui est en matière plastique ou en verre.

14. Procédé de fabrication du récipient (10) selon la revendication 7, comprenant les étapes consistant à appliquer une couche d'un activateur de coagulation intrinsèque (34) à une surface intérieure du récipient (10), appliquer un activateur de coagulation extrinsèque (36) sur la couche de l'activateur de coagulation intrinsèque (34).

15. Procédé selon la revendication 14, comprenant en outre l'étape consistant à appliquer une couche de tensioactif (32) avant d'appliquer la couche de l'activateur de coagulation intrinsèque (34).

16. Procédé selon la revendication 14, dans lequel l'activateur extrinsèque est choisi dans le groupe constitué par la thrombine, l'héparinase et le fibrinogène.

17. Procédé selon la revendication 14, dans lequel l'activateur intrinsèque est la silice.

18. Procédé selon la revendication 15, dans lequel le tensioactif est un tensioactif à base de silicone soluble dans l'eau.

19. Procédé selon la revendication 14, dans lequel l'étape d'application de la couche de l'activateur de coagulation intrinsèque (34) comprend le mélange de l'activateur de coagulation intrinsèque avec du polyvinylpyrrolidène, et la pulvérisation du mélange sur la surface interne, et dans lequel l'étape d'application de l'activateur de coagulation extrinsèque comprend le mélange de l'activateur de coagulation extrinsèque avec de l'eau, et la pulvérisation du mélange sur la couche de l'activateur de coagulation intrinsèque (34).

20. Procédé selon la revendication 14, dans lequel le récipient est en matière plastique ou en verre.

21. Réceptacle (50) imperméable à l'humidité, comprenant le récipient (10) selon l'une quelconque des revendications 7-13, et un matériau absorbant l'humidité (54).
